# EUROPEAN PATENT SPECIFICATION

(11) **EP 2 554 131 B1**
(45) Date of publication and mention of the grant of the patent: **12.08.2015**
(21) Application number: 12752416.3
(22) Date of filing: 27.02.2012
(51) Int. Cl.: A61B 18/12, A61B 1/00

(54) **TREATMENT TOOL FOR ENDOSCOPE**
ENDOSKOP-BEHANDLUNGSWERKZEUG
OUTIL DE TRAITEMENT POUR UN ENDOSCOPE

(30) Priority: 02.03.2011 JP 2011045031
(43) Date of publication of application: 06.02.2013
(73) Proprietor: OLYMPUS MEDICAL SYSTEMS CORP., Tokyo 151-0072 (JP)
(72) Inventor: SUZUKI, Keita, Tokyo 151-0072 (JP); KIMURA, Megumi, Tokyo 151-0072 (JP)
(74) Representative: Gunzelmann, Rainer
(86) International application number: PCT/JP2012/054777
(87) International publication number: WO 2012/118012

(56) References cited:
- WO-A1-2010/005006
- JP-A- 2000 271 132
- JP-A- 2000 271 132
- JP-A- 2010 017 224

## Description

### Technical Field

The present description relates to a treatment instrument for endoscope.

### Background Art

Conventionally, a treatment instrument for endoscope is known that is used to perform surgery using an endoscope. As an example of this type of treatment instrument for endoscope, for example, Patent Document 1 describes a treatment instrument wherein two operation wires (operation shaft members), the tips whereof are respectively connected to a pair of forceps members, can move relatively and are provided inside a sheath member formed into a cylindrical shape. With the treatment instrument described in Patent Document 1, the pair of forceps members can be operated by moving two operation wires forward and backward inside the sheath member.

Furthermore, Patent Document 2 describes a treatment instrument (high-frequency treatment instrument) wherein an operation wire for operating a pair of forceps members, and a power-supply wire for passing a high-frequency current through the pair of forceps members are each provided inside a sheath member (coil sheath). With the treatment instrument described in Patent Document 2, the pair of forceps members can be opened and closed by moving the operation wire forward and backward inside the sheath member.

### [Prior Art Document]

### [Patent Document]

Patent Document 1 : Japanese Unexamined Patent Application, First Publication No. 2005-187
Patent Document 2 : Japanese Unexamined Patent Application, First Publication No. 2010-17224

WO 2010/005006 A discloses a high-frequency treatment instrument used with high-frequency current supplied from a power supply provided with a treatment section body for treating a biomedical tissue, a conductive section which is provided, on a surface contacting with the biomedical tissue of the treatment section body, so as not to be electrically connected with the treatment section body, and a power supply means which is disposed so as to electrically connect the conductive section with the power supply such that a conductive outer surface is not exposed, and so as not to be electrically connected with the treatment section body.

### Summary of the Invention

### [Problems to be Solved by the Invention]

However, in the treatment instrument described in Patent Document 1 and Patent Document 2, two operation wires are arranged inside the sheath member, under a condition that outer peripheral surfaces of those two operation wires are capable of contacting each other. Therefore, when the pair of forceps members is operated, there will be cases where two operation wires are mutually slid.

Specifically, in the treatment instrument described in Patent Document 2, when the pair of forceps members is opened and closed, the operation wire and the power-supply wire slide.

In the case that the operation wire and the power-supply wire are mutually slid inside the sheath member, a frictional resistance is generated between the operation wire and the power-supply wire increases by an amount equivalent to the frictional resistance.

Therefore, in the treatment instrument described in Patent Document 1 and Patent Document 2, when the pair of forceps members is operated, the actuating force increases.

The present invention has as its object to provide a treatment instrument for endoscope that it is capable of operating even when an amount of the actuating force is small. The invention is as defined in the appended claims that follow. The embodiments, aspects or examples of the present description that do not fall within the scope of said claims are provided for illustration purposes only and do not form part of the present invention.

### [Means for Solving the Problem]

According to a first aspect of the present description a treatment instrument according to claim 1 is provided.

According to a second aspect of the present description, the first wire includes: a second core member, and a second covering member having a different surface condition from that of the first wire that covers the second core member.

According to a fourth aspect of the present description, outer peripheral surfaces of the first covering member and the second covering member have mutually different coefficients of friction.

According to a fifth aspect of the present description, the first covering member and the second covering member are made from different materials.

According to a sixth aspect of the description, in the treatment instrument for endoscope according to either of the fourth or fifth aspects, a first concavoconvex pattern is formed in the outer peripheral surface of the first covering member.

According to a seventh aspect of the description, in the treatment instrument for endoscope according to the sixth aspect, a second concavoconvex pattern having a different shape to that of the first concavoconvex pattern is formed in the outer peripheral surface of the second covering member, by a formation method that is different from that of the first concavoconvex pattern.

According to an eight aspect of the present description in the treatment instrument for endoscope according to any one of the first through seventh aspects, at least one of the first wire and the second wire has a conductive property.

According to a ninth aspect of the present description, in the treatment instrument for endoscope according to any one of the first through eighth aspects, on one of the first wire and the second wire, it has a conductive property and is capable of being electrically connected to a power-supply device supplying an electrical energy. On other of the first wire and the second wire, it is insulated to the one of the first wire and the second wire. The treatment instrument for endoscope further includes a treatment part and an operation part. The treatment part carries out medical treatment to a living tissue using the electrical energy that is supplied from the power-supply device via the one of the first wire and the second wire, the treatment part is electrically and mechanically connected to the one of the first wire and the second wire, and the treatment part is mechanically connected to the other of the first wire and the second wire. The operation part is mechanically connected to the other of the first wire and the second wire, and the operation part transmits a driving force to the treatment part via the other of the first wire and the second wire.

### [Effects of the Invention]

According to the treatment instrument for endoscope of the present description, since the surface condition of the outer peripheral surface of the first wire and the surface condition of the outer peripheral surface of the second wire are different, sliding resistance between the first wire and the second wire can be reduced. For this reason, this enables the treatment instrument for endoscope to be operated even with a small amount of actuating force.

### Brief Description of the Drawings

FIG. 1 is a side view showing a treatment instrument for endoscope in one embodiment of the present description.
FIG. 2 is a partial cross-sectional view showing the configuration of a treatment part vicinity of a treatment instrument for endoscope in one embodiment of the present description.
FIG. 3 is an explanatory view of an operation of a treatment part of a treatment instrument for endoscope in one embodiment of the present description.
FIG. 4 is a partial cross-sectional view of a treatment part of a treatment instrument for endoscope in one embodiment of the present description, and shows a view in a direction of an arrow A in FIG. 2.
FIG. 5 is a partial cross-sectional view of a treatment part of a treatment instrument for endoscope in one embodiment of the present description, and shows a view in a direction of an arrow B in FIG. 4.
FIG. 6 is a cross-sectional view taken along the line C-C in FIG. 4.
FIG. 7 is a view showing the configuration of a modified example (first modified example) of one embodiment of the present description, and shows a cross-sectional view taken along the line D-D in FIG. 6.
FIG. 8 is an enlarged view showing the section in FIG. 7 indicated by symbol X.
FIG. 9 is a view of another example configuration of a modified example (second modified example) of one embodiment of the present description, and shows an enlarged view of a section corresponding to the section in FIG. 7 indicated by symbol X.
FIG. 10 is a view showing another example configuration (fourth modified example) of a treatment instrument for endoscope in one embodiment of the present description, and shows an enlarged view of a section corresponding to the section in FIG. 7 indicated by symbol X.
FIG. 11 is a view showing yet another example configuration of a modified example (fifth modified example) of a treatment instrument for endoscope in one embodiment of the present description, and shows an enlarged view of a section corresponding to the section in FIG. 7 indicated by symbol X.

### Description of Embodiments

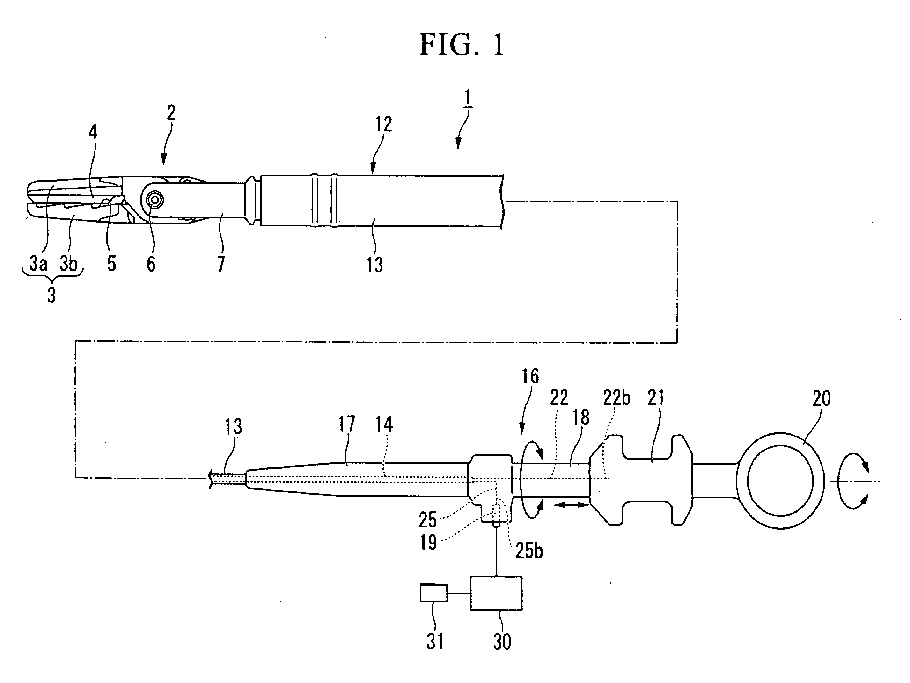

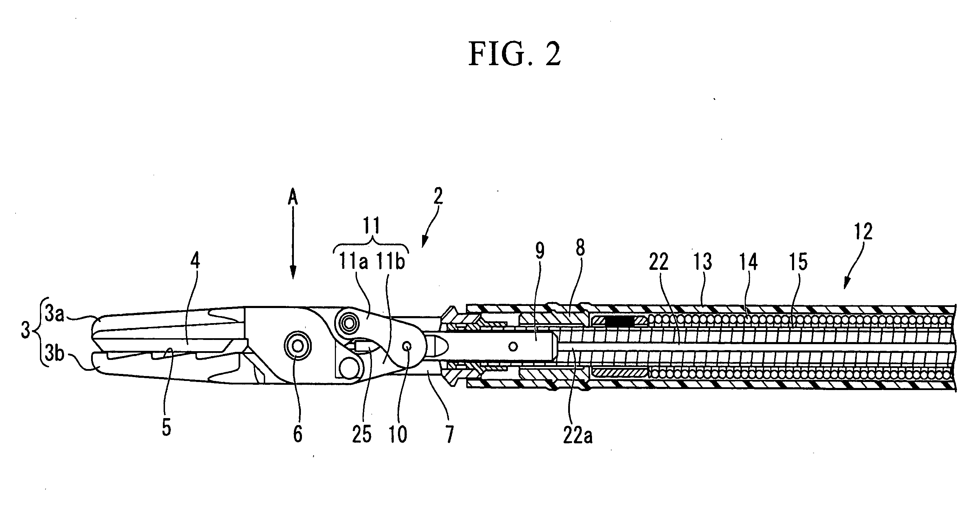

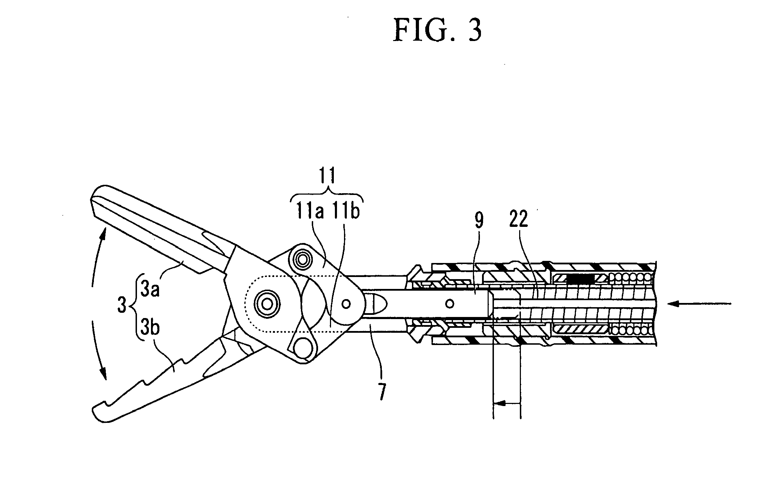

A treatment instrument for endoscope in one embodiment of the description will be explained. FIG. 1 is a side view showing a treatment instrument for endoscope 1 in this embodiment. FIG. 2 is a partial cross-sectional view showing the configuration of a treatment part 2 vicinity of the treatment instrument for endoscope 1. FIG. 3 is an explanatory view of an operation of the treatment part 2 of the treatment instrument for endoscope 1.

The treatment instrument for endoscope 1 of the embodiment is a treatment instrument for carrying out medical treatment to a living tissue. The treatment instrument for endoscope 1 is inserted into a treatment instrument channel of an endoscope, and used with the endoscope.

As shown in FIGS. 1 and 2, the treatment instrument for endoscope 1 includes a treatment part 2 for carrying out treatment to a living tissue, an elongated and flexible insertion part 12, the treatment part 2 being attached to the distal end of the insertion part 12, an operation part 16 provided at the proximal end of the insertion part 12, an operation wire 22 (first wire), and a power-supply wire 25 (second wire). The power-supply wire 25 is provided inside the insertion part 12.

As shown in FIG. 2, the treatment part 2 includes a pair of forceps members 3 (first forceps member 3a and second forceps member 3b) for gripping the living tissue, a transmitting member 9 fixed to the distal end of the operation wire 22, a pair of linking members 11 (first linking member 11a and second linking member 11b) for connecting the pair of forceps members 3 to the transmitting member 9, and a covering member 7 that supports the connecting rod 3 such that they can open and close.

The first forceps member 3a has a wire-shaped incision electrode 4 for passing a high-frequency current through the living tissue. The incision electrode 4 is electrically connected to the power-supply wire 25. The incision electrode 4 is configured such that a high-frequency current is supplied via the power-supply wire 25. The outer surface of the first forceps member 3a is coated with an insulating material, excepting the two points where it connects to the incision electrode 4 and to the connection point between the first forceps member 3a and the power-supply wire 25.

In the treatment instrument for endoscope 1 of this embodiment, the high-frequency current supplied to the incision electrode 4 flows through the body of the patient on whom the treatment instrument for endoscope 1 is being used to a return electrode 31 described below (see FIG. 1). That is, the treatment instrument for endoscope 1 of the embodiment is a so-called monopolar high-frequency treatment instrument.

The second forceps member 3b has a forceps face 5 with a saw-toothed shape that faces the incision electrode 4 of the first forceps member 3a.

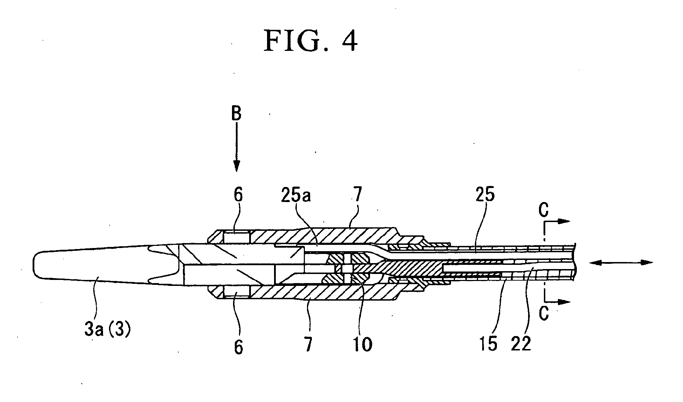

The first forceps member 3a and the second forceps member 3b are mutually rotatably connected at a connecting shaft part 6. Both ends of a connecting member are connected to the covering member 7 (see FIG. 4).

FIG. 4 is a partial cross-sectional view of the treatment instrument for endoscope 1, and shows a view in a direction of an arrow A of FIG. 2.

As shown in FIGS. 2 and 4, the covering member 7 is fixed to an inner coil sheath 15 described below. The covering member 7 is configured to be rotatable about the center axis of rotation of an outer sheath 13 constituting the outermost layer of the insertion part 12. Thus, when the inner coil sheath 15 is rotated around the center axis of rotation within the insertion part 12, the covering member 7 and the pair of forceps members 3 rotate in conjunction with the rotation of the inner coil sheath 15.

As shown in FIG. 2, a substantially cylindrical supporting member 8 is provided between the covering member 7 and the outer sheath 13. The supporting member 8 is fixed to the inner peripheral surface of the outer sheath 13, and the covering member 7 is rotatably inserted into the supporting member 8.

The transmitting member 9 is provided inside the covering member 7. One part of the proximal end of the transmitting member 9 is configured to be capable of being inserted inside the inner coil sheath 15 described below. The distal end of the transmitting member 9 is rotatably connected to the first linking member 11a and the second linking member 11b by a pin 10.

The first linking member 11a and the second linking member 11b are rotatably connected to the transmitting member 9 by the pin 10, and are rotatably connected to each of the first forceps member 3a and the second forceps member 3b. The first linking member 11a and the second linking member 11b convert the amount of actuating force (pressing force and traction force) that moves the operation wire 22 forward and backward in the direction of the center axis of the insertion part 12 to a driving force that opens and closes the pair of forceps members 3.

The insertion part 12 includes an outer sheath 13 formed from a tube-shaped resin, an outer coil sheath 14 inserted inside the outer sheath 13, and an inner coil sheath 15 inserted inside the outer coil sheath 14.

The outer sheath 13 is a member provided to the insertion part 12 with the aim of preventing liquid and the like from infiltrating inside the insertion part 12, and is flexible.

The outer coil sheath 14 is a sheath formed by winding a metal wire that is circular in cross-section into a coil shape, and is flexible.

The inner coil sheath 15 is a flat coil sheath formed by winding a metal wire that is rectangular in cross-section into a coil shape, and is flexible.

The distal end of the inner coil sheath 15 is fixed to the distal end of the outer coil sheath 14 by a method such as, for example, soldering or laser welding. Thus the inner coil sheath 15 and the outer coil sheath 14 integrally rotate with respect to the outer sheath 13.

In the embodiment, the outer coil sheath 14 and the inner coil sheath 15 are arranged coaxially within the outer sheath 13. It is thus possible to obtain both a resistance to compression in the direction of the center axis of the insertion part 12 and a rotational following performance in the center axis direction of the insertion part 12. Furthermore, the insertion part 12 is entirely flexible, and can be inserted into a treatment instrument channel of a flexible endoscope.

As shown in FIG. 1, the operation part 16 includes a cylindrical main-body part 17, a rod-shaped rotation-operation body 18, and a slider 21. The proximal ends of the outer coil sheath 14 and the inner coil sheath 15 are fixed to the rotation-operation body 18. The slider 21 is connected so that it can move forward and backward in the direction of the longitudinal axis of the rotation-operation body 18.

The proximal end of the outer sheath 13 is fixed to the main-body part 17. In addition, the outer coil sheath 14 and the inner coil sheath 15 are rotatably inserted inside the main-body part 17.

The rotation-operation body 18 is rotatably connected to the main-body part 17. The rotation-operation body 18 can rotate the outer coil sheath 14, the inner coil sheath 15, the operation wire 22, and the power-supply wire 25 shown in FIG. 2 integrally with respect to the main-body part 17. As shown in FIG. 1, the rotation-operation body 18 is provided with a terminal part 19 which can be connected to a high-frequency power source device 30 (power-supply device). The high-frequency power source device 30 supplies high-frequency current (electrical energy) to the power-supply wire 25. The proximal end 25b of the power-supply wire 25 is fixed to the terminal part 19. A ring-shaped finger-hook part 20 for enabling a user of the treatment instrument for endoscope 1 to hook his fingers is formed at the proximal end of the rotation-operation body 18.

In the embodiment, the high-frequency power source device 30 includes a return electrode 31 used by attaching it to the body-surface of the patient on whom the treatment instrument for endoscope 1 is to be used. The high-frequency current supplied to the incision electrode 4 of the treatment instrument for endoscope 1 thus flows through the body of the patient to the return electrode 31.

The outer surface of the slider 21 is formed with a concave shape so that the user of the treatment instrument for endoscope 1 can hook his fingers around it. The proximal end 22b of the operation wire 22 is fixed to the slider 21. The slider 21 moves in the direction of the longitudinal axis of the rotation-operation body 18 by an operation of the user. As a result, the force that the user applies to the slider 21 is transmitted to the operation wire 22, and the operation wire 22 moves forward and backward in the direction of its center axis.

In the operation part 16, when the slider 21 moves forward and backward with respect to the rotation-operation body 18, a driving force for opening and closing the pair of forceps members 3 is transmitted through the operation wire 22 to the treatment part 2. In the embodiment, the force that the user applies to the slider 21 when he moves it forward and backward is the amount of actuating force in the treatment instrument for endoscope 1.

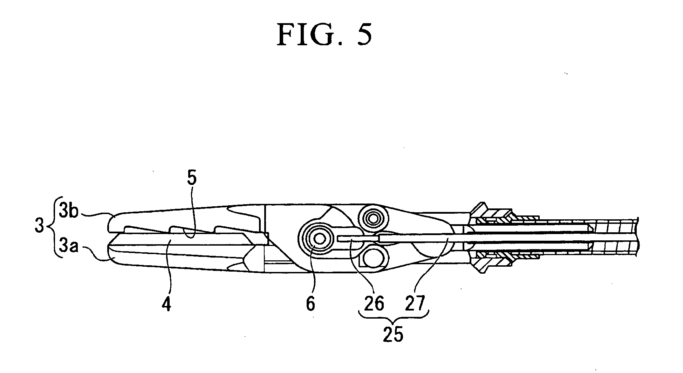

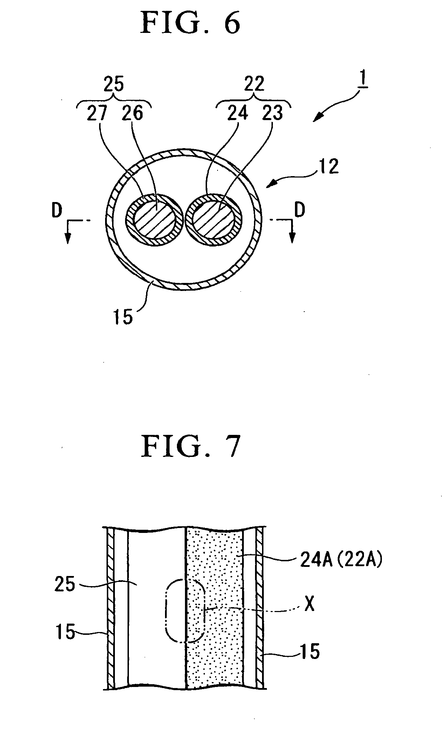

FIG. 5 is a partial cross-sectional view of the treatment instrument for endoscope 1, and shows a view in a direction of an arrow B in FIG. 4. FIG. 6 is a cross-sectional view taken along the line C-C in FIG. 4.

As shown in FIGS. 2 and 6, the operation wire 22 and the power-supply wire 25 are disposed substantially parallel with each other inside the inner coil sheath 15 of the insertion part 12. In the embodiment, to reduce the outer diameter of the insertion part 12, the inner coil sheath 15 does not include partitions for partitioning the operation wire 22, the power-supply wire 25, and a storage space. Therefore, the outer peripheral surface of the operation wire 22 and the outer peripheral surface of the power-supply wire 25 can touch each other inside the inner coil sheath 15.

The distal end 22a of the operation wire 22 is fixed to the transmitting member 9 of the treatment part 2 (see FIG. 2). The proximal end 22b of the operation wire 22 is fixed to the slider 21 of the operation part 16 (see FIG. 1). The operation wire 22 includes a first core member 23 formed into a wire shape, and an insulating first covering member 24 that covers the outer peripheral surface of the first core member 23.

As the material for the first core member 23 it is possible to use a flexible material that has little elongation when pulled in the direction of its center axis, and high resistance to compression when pressed in the direction of its center axis. For example, a thin wire material or the like of metal or alloy can be used as the first core member 23.

The first covering member 24 is formed from an insulating material. For example, polyethylene (PE), PE elastomer, polyetheretherketone (PEEK), or fluorine resin can be used as the material for the first insulating member 24. As specific examples of fluorine resin, for example, polytetrafluoroethylene (PTFE), perfluoroalkoxy alkane (PFA), perfluoro ethylene-propylene copolymer (FEP), and the like, can be used.

As shown in FIGS. 1 and 4, the distal end 25a of the power-supply wire 25 is fixed to the first forceps member 3a of the treatment part 2. The proximal end 25b of the power-supply wire 25 is fixed to the terminal part 19 of the operation part 16.

As shown in FIGS. 5 and 6, the power-supply wire 25 includes a second core member 26 made from a conductor, and an insulating second covering member 27 that covers the outer peripheral surface of the second core member 26. In this embodiment, the insulating second covering member 27 insulates the power-supply wire 25 from the operation wire 22. Since the first covering member 24 also has an insulating effect, it further increases the insulation between the power-supply wire 25 and the operation wire 22.

As the material for the second core member 26, a material that allows a high-frequency current to be conducted can be used. For example, a thin wire material of metal or alloy can be used as the second core member 26. In the embodiment, the second core member 26 of the power-supply wire 25 is fixed to the treatment part 2 and the terminal part 19, and a high-frequency current can be supplied from the terminal part 19 and through the second core member 26 to the incision electrode 4 of the first forceps member 3a.

The second covering member 27 is made from a different type of material from that of the first covering member 24. The material for the second covering member 27 can be determined by selecting a different one from that used for the first covering member 24 from among polyethylene (PE), PE elastomer, polyetheretherketone (PEEK), and fluorine resin. That is, the combination of materials for the first covering member 24 and the second covering member 27 is selected such that, after the first core member 23 and the second core member 26 have been covered with the first covering member 24 and the second covering member 27 respectively, the surface conditions of the first covering member 24 and the second covering member 27 are mutually different.

Due to the mutually different surface conditions of the first covering member 24 and the second covering member 27, the coefficient of friction between the outer peripheral surface of the first covering member 24 and the outer peripheral surface of the second covering member 27 is different from the coefficient of friction between first covering members 24 and the coefficient of friction between second covering members 27.

In the embodiment, the distal end 25a of the power-supply wire 25 is connected via the first forceps member 3a and the covering member 7 to the distal end of the insertion part 12. The proximal end 25b of the power-supply wire 25 is fixed to the rotation-operation body 18 of the operation part 16. Consequently, while the operation wire 22 can move forward and backward within the insertion part 12, the power-supply wire 25 cannot do so. Since the slider 21 is moved forward and backward with respect to the rotation-operation body 18 in this manner, the operation wire 22 and the power-supply wire 25 move relatively.

Subsequently, the effects of the treatment instrument for endoscope 1 will be explained.

When using the treatment instrument for endoscope 1, the user moves the slider 21 of the operation part 16 shown in FIG. 1 forward and backward with respect to the rotation-operation body 18. An actuating force is thereby applied via the operation wire 22 to the pair of forceps members 3. When the operation wire 22 is moved to the distal-end side of the insertion part 12 while the pair of forceps members 3 are in the closed state, the pair of forceps members 3 will open (see FIG. 3). Conversely, when the operation wire 22 is moved to the proximal-end side of the insertion part 12 while the pair of forceps members 3 are in the open state, the pair of forceps members 3 will close.

The power-supply wire 25 fixed to the first forceps member 3a, which is the one of the pair of forceps members 3 where the incision electrode 4 is formed, does not move within the inner coil sheath 15 even if the pair of forceps members 3 open and close.

Therefore, if the operation wire 22 for opening and closing the pair of forceps members 3 is moved forward and backward within the inner coil sheath 15, the operation wire 22 and the power-supply wire 25 move relatively. At this time, the outer peripheral surface of the operation wire 22 and the outer peripheral surface of the power-supply wire 25 slide.

In this embodiment, the covering member of the operation wire 22 and the covering member of the power-supply wire 25 are made from different materials. The surface condition of the outer peripheral surface of the operation wire 22 and the surface condition of the outer peripheral surface of the power-supply wire 25 are mutually different due to the materials they are made from. That is, in this embodiment, the microscopic structure of the outer peripheral surface of the operation wire 22 and the microscopic structure of the outer peripheral surface of the power-supply wire 25 are mutually different.

For example, when the outer peripheral surface of the operation wire 22 and the outer peripheral surface of the power-supply wire 25 are made from the same material and formed in similar shapes, the microscopic structure of the outer peripheral surface of the operation wire 22 and the microscopic structure of the outer peripheral surface of the power-supply wire 25 will be substantially similar. In such a case, when the operation wire 22 and the power-supply wire 25 touch each other, there will be cases where the outer peripheral surface of the operation wire 22 and the outer peripheral surface of the power-supply wire 25 fit together or closely attach.

When the outer peripheral surface of the operation wire 22 and the outer peripheral surface of the power-supply wire 25 fit together or closely attach, the amount of actuating force required to open and close the pair of forceps members 3 by moving the operation wire 22 forward and backward is greater than when the operation wire 22 and the power-supply wire 25 are not touching each other. Specifically, the amount of actuating force increases by an amount equivalent to the frictional resistance between the covering member of the operation wire 22 and the covering member of the power-supply wire 25.

In contrast, in the embodiment, the outer peripheral surface of the operation wire 22 and the outer peripheral surface of the power-supply wire 25 have mutually different microscopic structures. Therefore, when the outer peripheral surfaces of the operation wire 22 and the power-supply wire 25 make contact with each other, a plurality of microscopic gaps are formed between them along their entire lengths. This configuration makes it more difficult for the outer peripheral surfaces of the operation wire 22 and the power-supply wire 25 to fit together or closely attach.

As a result, since the first covering member 24 of the operation wire 22 and the second covering member 27 of the power-supply wire 25 are made from mutually different materials, even if the operation wire 22 and the power-supply wire 25 touch each other, frictional resistance between the operation wire 22 and the power-supply wire 25 can be reduced. This makes it possible to move the operation wire 22 forward and backward with a smaller amount of actuating force than when the covering members of the operation wire 22 and the power-supply wire 25 are made from the same material.

Also, since the first covering member 24 and the second covering member 27 are made from different materials, there is less fictional force between their outer surfaces.

In this way, according to the treatment instrument for endoscope 1 of the embodiment, since the surface condition of the outer peripheral surface of the operation wire 22 and the surface condition of the outer peripheral surface of the power-supply wire 25 are different, sliding resistance between the operation wire 22 and the power-supply wire 25 can be reduced. This enables the treatment instrument for endoscope 1 to be operated even with a low amount of actuating force.

Since the operation wire 22 is formed by covering the first core member 23 with the first covering member 24, and the power-supply wire 25 is formed by covering the second core member 26 with the second covering member 27, there is high insulation between the operation wire 22 and the power-supply wire 25.

Due to the different microscopic structures of the outer peripheral surfaces of the first covering member 24 and the second covering member 27, their coefficients of friction are mutually different, as already mentioned. This can reduce the possibility that the outer peripheral surface of the first covering member 24 and the outer peripheral surface of the second covering member 27 will fit together or closely attach.

Furthermore, since the material of the first covering member 24 and the material of the second covering member 27 are mutually different, it is easy to configure covering members with mutually different surface conditions.

### [Modified Examples]

Subsequently, a first modified example of the treatment instrument for endoscope 1 of the embodiment will be explained.

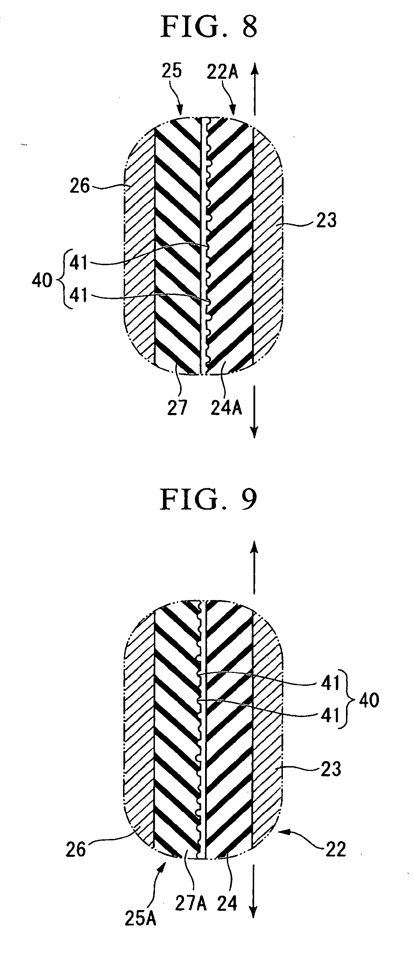

FIG. 7 is a view showing the configuration of one part of the treatment instrument for endoscope 1 of the modified example, and shows a cross-sectional view taken along the line D-D in FIG. 6. FIG. 8 is an enlarged view showing the section in FIG. 7 indicated by symbol X.

As shown in FIG. 7, a point of difference in the modified example is that, instead of the operation wire 22, an operation wire 22A is provided.

The operation wire 22A includes a first core member 23 and a first covering member 24A. The first covering member 24A differs from the first covering member 24 described above in that a first concavoconvex pattern 40 is formed in its outer peripheral surface. The first concavoconvex pattern 40 is formed by a method such as, for example, emboss processing during the step of covering the first core member 23 with the first covering member 24A. The first concavoconvex pattern 40 of this embodiment has a plurality of dimples 41 that are indented radially inward from the outer peripheral surface of the first covering member 24. The depth of the dimples 41 of the first concavoconvex pattern 40 is adjusted at the time of molding so that they do not form holes exposing the first core member 23 in the first covering member 24.

As shown in FIG. 8, in the modified example, the first concavoconvex pattern 40 is formed in the outer peripheral surface of the operation wire 22A. Thus, even if the outer peripheral surface of the operation wire 22A contacts the outer peripheral surface of the power-supply wire 25, at the dimples 41, gaps are formed between the outer peripheral surface of the operation wire 22A and the outer peripheral surface of the power-supply wire 25. The treatment part 2 can therefore be operated with a small amount of actuating force, as in the treatment instrument for endoscope 1 described above, without the outer peripheral surfaces of the operation wire 22A and the power-supply wire 25 closely attaching with each other.

In the modified example, since the dimples 41 forming the first concavoconvex pattern 40 change the frictional resistance between the outer peripheral surfaces of the first covering member 24A and the second covering member 27, the first covering member 24A and the second covering member 27 can be made from the same material.

FIG. 9 is a view of another configuration of the modified example described above (second modified example), and shows an enlarged view of a section corresponding to the section in FIG. 7 indicated by symbol X.

As shown in FIG. 9, when the operation wire 22 described in the embodiment is combined with a power-supply wire 25A including a second covering member 27A with the first concavoconvex pattern 40 of the modified example formed therein, similar effects to those of the modified example are obtained.

As yet another example configuration of the modified example described above (third modified example), two mutually different types of concavoconvex patterns (a first concavoconvex pattern and a second concavoconvex pattern) can be formed in the outer peripheral surfaces of the operation wire 22A and the power-supply wire 25.

For example, the first covering member 24 with a first concavoconvex pattern and the second covering member 27 with a second concavoconvex pattern can be formed on the operation wire 22 and the power-supply wire 25 using mutually different formation methods, so that the apertures of their dimples and the densities of their pluralities of dimples are different.

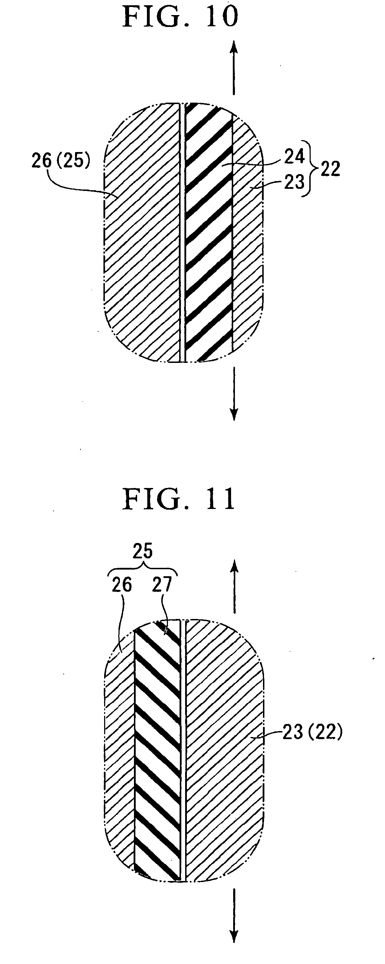

FIG. 10 is a view showing another example configuration (fourth modified example) of the treatment instrument for endoscope 1, and shows an enlarged view of a section corresponding to the section in FIG. 7 indicated by symbol X.

FIG. 11 is a view of yet another example configuration of a modified example (fifth modified example) of the treatment instrument for endoscope 1, and shows an enlarged view of a section corresponding to the section of FIG. 7 indicated by symbol X.

As shown in FIGS. 10 and 11, even if only one of the operation wire 22 and the power-supply wire 25 is covered, the surface conditions of the outer peripheral surface of the operation wire 22 and the outer peripheral surface of the power-supply wire 25 are different, and similar effects to those of the embodiment and modified examples described above are obtained.

While the embodiment described above is an example of a monopolar configuration including the operation wire 22 that current need not be passed through and the power-supply wire 25 that the high-frequency current is passed through, the treatment instrument for endoscope of this embodiment is not limited to a monopolar configuration. For example, the present invention can be favorably applied in a bipolar treatment instrument for endoscope, in which a pair of power-supply wires that high-frequency current is passed through are arranged within an insertion part, obtaining the effects of the invention described above.

While the embodiment describes an example where two wires are arranged inside a cylindrical member, three or more wires may be arranged inside one cylindrical member. In that case, the effects of the invention can be obtained by ensuring that those of the outer peripheral surfaces of the three or more wires that can contact each other have mutually different surface conditions.

The constituent elements of the embodiment and the modified examples described above can be combined as appropriate.

### Industrial Applicability

According to the treatment instrument for endoscope of the present description since the surface condition of the outer peripheral surface of the first wire and the surface condition of the outer peripheral surface of the second wire are different, sliding resistance between the first wire and the second wire can be reduced.

### Reference Signs List

- 1: treatment instrument for endoscope
- 2: treatment part
- 3: forceps members
- 4: incision electrode
- 12: insertion part (cylindrical member)
- 13: outer sheath
- 14: outer coil sheath
- 15: inner coil sheath
- 16: operation part
- 21: slider
- 22, 22A: operation wire
- 23: first core member
- 24, 24A: first covering member
- 25 25A: power-supply wire
- 26: second core member
- 27: second covering member
- 40: first concavoconvex pattern

The present invention is as set out in the appended claims that follow.

## Claims

1. A treatment instrument for an endoscope comprising:
a sheath (15) that is capable of being inserted into a treatment instrument (1) channel of an endoscope;
a treatment part (2) provided on a distal-end side of the sheath (15);
a first wire (22) that is connected to the treatment part (2) and inserted into the sheath (15), the first wire (22) comprising a first core member (23) and a first covering member (24) that covers the first core member (23); and
a second wire (25) that drives the treatment part (2), is connected to the treatment part (2) and is inserted into the sheath (15) so that the second wire (25) moves relative to the first wire (22) when the treatment part is actuated, in a state where a second outer peripheral surface of the second wire (25) contacts a first outer peripheral surface of the first wire (22),
**characterized in that**
the second wire includes a second core member (26) and a second covering member (27) that covers the second core member (26),
the first covering member (24) is made from a first material,
the second covering member (27) is made from a second material that is different from the first material, and
the coefficient of friction between the first outer peripheral surface of the first covering member (24) and the second outer peripheral surface of the second covering member (27) is different from the coefficient of friction between first covering members (24) and the coefficient of friction between second covering members (27).

2. The treatment instrument for an endoscope according to claim 1, wherein
the second wire (25) is a power-supply wire (25) that supplies electrical power to the treatment part (2).

3. The treatment instrument for an endoscope according to claim 1, wherein
a first concavoconvex pattern (40) that is defined as having a plurality of dimples that are indented radially inward from the outer peripheral surface of the first covering member (24) is formed in the outer peripheral surface of the first covering member (24).

4. The treatment instrument for an endoscope according to claim 3, wherein two mutually different types of concavoconvex patterns are formed in the outer peripheral surfaces of the first wire (22) and the second wire (25).

5. The treatment instrument for an endoscope according to claim 2, wherein
the first covering member (24) is made from a material having an electrical insulating effect.

6. The treatment instrument for an endoscope according to claim 1, wherein
a first outer peripheral surface of the first covering member (24) and a second outer peripheral surface of the second covering member (27) have mutually different microscopic structures.

## Patentansprüche

1. Behandlungsgerät für ein Endoskop, das umfasst:
eine Hülse (15), die dazu in der Lage ist, in einen
Behandlungsinstrumentenkanal (1) eines Endoskops eingeführt zu werden;
ein Behandlungsteil (2), das an einer distalen Endseite der Hülse (15) vorgesehen ist;
einen ersten Draht (22), der mit dem Behandlungsteil (2) verbunden und in die Hülse (15) eingeführt ist, wobei der erste Draht (22) ein erstes Kernelement (23) und ein erstes Abdeckungselement (24) umfasst, das das erste Kernelement (23) abdeckt; und
einen zweiten Draht (25), der das Behandlungsteil (2) antreibt, mit dem Behandlungsteil (2) verbunden ist und so in die Hülse (15) eingeführt ist, dass sich in einem Zustand, in dem eine zweite Außenumfangsfläche des zweiten Drahts (25) eine erste Außenumfangsfläche des ersten Drahts (22) berührt, der zweite Draht (25) relativ zu dem ersten Draht (22) bewegt, wenn das Behandlungsteil betätigt wird,
**dadurch gekennzeichnet, dass**
der zweite Draht ein zweites Kernelement (26) und ein zweites Abdeckungselement (27) umfasst, das das zweite Kernelement (26) abdeckt,
das erste Abdeckungselement (24) aus einem ersten Material besteht,
das zweite Abdeckungselement (27) aus einem zweiten Material besteht, das sich von dem ersten Material unterscheidet, und
der Reibungskoeffizient zwischen der ersten Außenumfangsfläche des ersten Abdeckungselements (24) und der zweiten Außenumfangsfläche des zweiten Abdeckungselements (27) sich von dem Reibungskoeffizienten zwischen ersten Abdeckungselementen (24) und dem Reibungskoeffizienten zwischen zweiten Abdeckungselementen (27) unterscheidet.

2. Behandlungsgerät für ein Endoskop gemäß Anspruch 1, bei dem der zweite Draht (25) ein Stromzufuhr-Draht (25) ist, der dem Behandlungsteil (2) elektrischen Strom zuführt.

3. Behandlungsgerät für ein Endoskop gemäß Anspruch 1, bei dem
ein erstes konkavkonvexes Muster (40), das als eine Mehrzahl von Vertiefungen umfassend definiert ist, die radial einwärts von der Außenumfangsfläche des ersten Abdeckungselements (24) eingekerbt sind, in der Außenumfangsfläche des ersten Abdeckungselements (24) ausgebildet ist.

4. Behandlungsgerät für ein Endoskop gemäß Anspruch 3, bei dem zwei wechselseitig verschiedene Typen von konkavkonvexen Mustern in den Außenumfangsfilächen des ersten Drahts (22) und des zweiten Drahts (25) ausgebildet sind.

5. Behandlungsgerät für ein Endoskop gemäß Anspruch 2, bei dem
das erste Abdeckungselement (24) aus einem Material besteht, das eine elektrisch isolierende Wirkung hat.

6. Behandlungsgerät für ein Endoskop gemäß Anspruch 1, bei dem
eine erste Außenumfangsfläche des ersten Abdeckungselements (24) und eine zweite Außenumfangsfläche des zweiten Abdeckungselements (27) wechselseitig verschiedene mikroskopische Strukturen haben.

## Revendications

1. Instrument de traitement pour un endoscope comprenant :
une gaine (15) qui est capable d'être insérée dans un canal d'instrument de traitement (1) d'un endoscope ;
une partie de traitement (2) prévue sur un côté d'extrémité distale de la gaine (15) ;
un premier câble (22) qui est connecté à la partie de traitement (2) et inséré dans la gaine (15), le premier câble (22) comprenant un premier élément d'âme (23) et un premier élément couvrant (24) qui couvre le premier élément d'âme (23) ; et
un deuxième câble (25) qui entraîne la partie de traitement (2) est connecté à la partie de traitement (2) et est inséré dans la gaine (15) de sorte que le deuxième câble (25) se déplace par rapport au premier câble (22) lorsque la partie de traitement est actionnée dans un état dans lequel une deuxième surface périphérique externe du deuxième câble (25) contacte une première surface périphérique externe du premier câble (22),
**caractérisé en ce que**
le deuxième câble comprend un deuxième élément d'âme (26) et un deuxième élément couvrant (27) qui couvre le deuxième élément d'âme (26),
le premier élément couvrant (24) est composé d'un premier matériau,
le deuxième élément couvrant (27) est composé d'un deuxième matériau qui est différent du premier matériau, et
le coefficient de frottement entre la première surface périphérique externe du premier élément couvrant (24) et la deuxième surface périphérique externe du deuxième élément couvrant (27) est différent du coefficient de frottement entre les premiers éléments couvrants (24) et les deuxièmes éléments couvrants (27).

2. Instrument de traitement pour un endoscope selon la revendication 1, dans lequel
le deuxième câble (25) est un câble d'alimentation (25) qui délivre la puissance électrique à la partie de traitement (2).

3. Instrument de traitement pour un endoscope selon la revendication 1, dans lequel
un premier motif concavoconvexe (40) qui est défini comme ayant une pluralité de fossettes qui sont dentelées radialement vers l'intérieur en partant de la surface périphérique externe du premier élément couvrant (24) est formé dans la surface périphérique externe du premier élément couvrant (24).

4. Instrument de traitement pour un endoscope selon la revendication 3, dans lequel deux types mutuellement différents de motifs concavoconvexes sont formés dans les surfaces périphériques externes du premier câble (22) et du deuxième câble (25).

5. Instrument de traitement pour un endoscope selon la revendication 2, dans lequel
le premier élément couvrant (24) est composé d'un matériau possédant un effet électriquement isolant.

6. Instrument de traitement pour un endoscope selon la revendication 1, dans lequel
une première surface périphérique externe du premier élément couvrant (24) et une deuxième surface périphérique externe du deuxième élément couvrant (27) présentent des structures microscopiques mutuellement différentes.
